(19) Europäisches Patentamt
European Patent Office
Office européen des brevets

(11) Publication number: **0 239 739**
**B1**

(12) **EUROPEAN PATENT SPECIFICATION**

(45) Date of publication of patent specification: **29.08.90**

(51) Int. Cl.$^5$: **C 07 C 9/10,** C 07 C 2/76 // B01J29/28

(21) Application number: **87101269.6**

(22) Date of filing: **30.01.87**

(54) **Production of butanes from propane.**

(30) Priority: **28.03.86 US 845284**

(43) Date of publication of application:
**07.10.87 Bulletin 87/41**

(45) Publication of the grant of the patent:
**29.08.90 Bulletin 90/35**

(84) Designated Contracting States:
**BE DE FR GB IT NL**

(56) References cited:
**GB-A-2 091 754**
**US-A-4 070 411**
**US-A-4 490 569**

(73) Proprietor: **MOBIL OIL CORPORATION**
**3224 Gallows Road**
**Fairfax Virginia 22037-0001 (US)**

(72) Inventor: **Morrison, Roger Allan**
**57 Juniper Court**
**Beptford New Jersey 08096 (US)**

(74) Representative: **Kador & Partner**
**Corneliusstrasse 15**
**D-8000 München 5 (DE)**

**EP 0 239 739 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned with the production of butanes from propane.

Zeolite ZSM—5, described in US Patent 3,702,886, is a shape-selective zeolite having an intermediate pore size, i.e., a pore size smaller than Zeolite X or Y but larger than Zeolite A. Since its discovery, ZSM—5 and related zeolites have been shown to be capable of efficiently catalyzing some unusual hydrocarbon (and other) conversions. US 4,120,910 to Chu describes the conversion of ethane to aromatic hydrocarbons at 600°C. At lower temperatures, such as at 343°C (650°F) the higher linear and singly methyl-branched alkanes having six or more carbon atoms react with benzene to form alkylbenzenes and lower alkanes, presumably by reaction of olefins formed in situ from the alkane (by acid cracking) with the benzene.

Alkanes having eight or more carbon atoms are easily cracked with an acidic catalyst. The reaction proceeds with little disproportionation to paraffins of higher molecular weight than the charge. It is known that as the molecular weight of the charged paraffin decreases, the reactivity of the paraffin also decreases.

Accordingly, the present invention provides a process for producing butanes from propane characterized by contacting a propane feed in the absence of added hydrogen with a catalyst comprising a crystalline zeolite having a silica-to-alumina ratio of at least 12 and a Constraint Index of 1 to 12 at a temperature of 260 to 482°C (500 to 900°F), a pressure of 450 to 10400 kPa (50 to 1500 psig), and a WHSV of 0.1 to 10 whereby up to 25% of the propane feed is converted with a selectivity to butanes of at least 35 wt.%.

This invention allows the conversion of propane by-product, which has a low economic value and is often burned for fuel, to more valuable high-octane alkylate for blending in gasoline.

The process of this invention is simple. Only liquid propane need be charged to a reactor. Reactor effluent may be separated conventionally to recover unreacted propane for recycle. The catalyst may be in a simple fixed bed, although a fluidized bed may be used. No hydrogen is required, and indeed is detrimental. The catalyst lasts a long time so frequent regeneration is not needed.

The propane feed should have a propane content of at least 95 wt%. Suitable sources of propane include refinery streams and natural gas liquids.

The catalytic conversion is effected under a temperature, pressure, and weight hourly space velocity (WHSV) effective to convert up to 25% of the propane feed, with a selectivity to butanes of at least 35 wt%, preferably 45 to 95 wt%, with the remainder being converted principally to other C1 to C5 alkanes. Increasing temperature, or pressure, or decreasing space velocity increases conversion. Many combinations of T, P and WHSV will work. It is difficult to specify operable ranges for the three variables. In general the individual parameters fall within the ranges shown below:

| | Broad | Preferred |
|---|---|---|
| Temperature, | 500—900°F | 600—800°F |
| | 260—482°C | 316—427°C |
| Pressure, psig | 50—1500 | 400—1000 |
| kPa | 450—10400 | 2900—7000 |
| WHSV | 0.1 to 10 | 0.4 to 5.0 |

Within these constraints, useful yields of isobutane per pass are achieved without rapid catalyst aging.

The zeolites catalysts have an effective pore size of 5 to 8 angstroms, to freely sorb normal hexane. The zeolite must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not suitable. 10-membered rings are preferred, although excessive puckering of the rings or pore blockage may render these zeolites ineffective.

Most 12-membered rings do not offer sufficient constraint to work but the puckered 12-ring structure of TMA offretite shows some constrained access. Other 12-ring structures may exist which work.

A convenient measure of constrained access is the Constraint Index, C.I., of the zeolite. Zeolites which provide a highly restricted access to and egress from internal structure have a high Constraint Index. These zeolites usually have pores of small size, e.g. less than 5 angstroms. Zeolites which provide relatively free access to the internal zeolite structure have a low Constraint Index, and usually have pores greater than 8 angstroms. Constraint Index is described fully in U.S. Patent No. 4,016,218. The C.I. is determined with the hydrogen form of the zeolite, but that the C.I. is believed to be an attribute of the crystal structure.

C.I. values for some typical materials are shown in Table A.

TABLE A

| ZEOLITE | CI | (at test temperature) |
|---|---|---|
| ZSM-4 | 0.5 | (316°C) |
| ZSM-5 | 6-8.3 | (371°C-316°C) |
| ZSM-11 | 5-8.7 | (371°C-316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-38 | 2 | (510°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

The C.I. defines those zeolites which are useful in the instant invention. CI varies with severity of operations (conversion), the presence or absence of binders, crystal size, and the presence of occluded contaminants, etc. This explains the range of CI for ZSM—5, ZSM—11 and Zeolite Beta.

Preferred zeolites are ZSM—5, ZSM—11, ZSM—12, ZSM—22, ZSM—23, ZSM—35, ZSM—38, ZSM—48, ZSM—50, and similar materials.

U.S. Patent 3,702,886 describes ZSM—5.

ZSM—11 is described in U.S. Patent 3,709,979.

ZSM—12 is described in U.S. Patent 3,832,449.

ZSM—22 is described in U.S. Patent 4,046,859.

ZSM—23 is described in U.S. Patent 4,076,842.

ZSM—35 is described in U.S. Patent 4,016,245.

ZSM—38 is described in U.S. Patent 4,046,859.

ZSM—48 is described in U.S. Patent 4,397,827.

ZSM—50 is characterized, in terms of moles of oxides per 100 moles of silica on an anhydrous basis, as follows:

$$(0—10)M_{2/n}O:(1—5)Al_2O_3:(100)SiO_2$$

wherein m is at least one cation having a valence n, and wherein the zeolite is characterized by a distinctive X-ray diffraction pattern substantially as shown in Table B.

TABLE B

| Interplanar d-Spacing (A) | Relative Intensity, $I/I_o$ |
|---|---|
| 20.1 ± .03 | W |
| 11.1 ± .17 | S |
| 10.1 ± .16 | M |
| 9.7 ± .14 | W |
| 5.77 ± .09 | W |
| 5.61 ± .09 | W |
| 4.64 ± .07 | M |
| 4.35 ± .07 | M |
| 4.30 ± .07 | VS |
| 4.00 ± .06 | S |
| 3.85 ± .06 | M |
| 3.70 ± .06 | M |
| 3.42 ± .05 | W |
| 3.35 ± .05 | W |
| 3.27 ± .05 | M |
| 3.24 ± .05 | W |
| 2.94 ± .04 | W |
| 2.53 ± .04 | W |

These values were determined by standard techniques. As synthesized ZSM—50 has a formula, on an anhydrous basis and in terms of moles of oxides per 100 moles of silica, as follows:

$$(0—4)R_2O:(0—10)M_{2/n}O:(1—5)Al_2O_3:(100)SiO_2$$

wherein M is an alkali or alkaline earth metal, n is the valence of M, and R is an organic cation of a diquaternary directing agent compound generally expressed by the following formula:

$$X(CH_3)_3N(CH_2)_6N(CH_3)_3X$$

wherein X is an anion, e.g. halide, such as iodide.

Zeolite ZSM—50 can be prepared from a reaction mixture containing sources of an alkali or alkaline earth metal oxide, an oxide of aluminum, an oxide of silicon, an organic cation and water and having a composition, in terms of mole ratios of oxides, within the following ranges:

| Reactants | Useful | Preferred |
|---|---|---|
| $SiO_2/Al_2O_3$ | 20—100 | 30—90 |
| $OH^-/SiO_2$ | 0.1—0.6 | 0.1—0.3 |
| $R/SiO_2$ | 0.05—0.6 | 0.1—0.3 |
| $M/SiO_2$ | 0.01—1.0 | 0.1—0.6 |

wherein M is an alkali or alkaline earth metal and R is an organic cation derived from the above-identified diquaternary directing agent compound.

Zeolite ZSM—50 can be crystallized at either static or stirred condition in a suitable reactor vessel, such as polypropylene jars or teflon lined or stainless steel autoclaves. The usual temperature for crystallization is 100°C to 200°C for 48 hours to 15 days. Thereafter, the crystals are separated from the liquid and recovered.

ZSM—5 is the particularly preferred zeolite, characterized by high activity and favorable selectivity for isobutane.

It is preferred to use zeolites having high acid activity. Lower silica-to-alumina ratio are usually preferred, since the acid activity, as measured by the "alpha test", of these is usually higher. The "alpha test", a measure of the relative rate constant of the zeolite for cracking normal hexane is described by Miale et al. in Journal of Catalysis, Volume 6, No. 2, October 1966.

Zeolites, when prepared in the presence of organic cations, are not very active, possibly because the intra-crystalline free space is occupied by organic species from the forming solution. These organic templates may be removed by e.g., heating to 538°C in an inert atmosphere, base exchange with ammonium salts, then calcination at 538°C in air. If binder is used the activation step may be conducted after compositing the zeolite with the binder.

The zeolite can be used neat or with a binder. For better particle properties, or for ease of formation of shaped particles, however, a binder, such as alumina. Extrudate particles of 65 wt% zeolite and 35 wt% alumina binder work very well.

The zeolite is preferably converted to the hydrogen form prior to use by conventional techniques. As used herein, H—ZSM—5, H—ZSM—50, etc. refer to the zeolites in the hydrogen form and without a hydrogenation-dehydrogenation (H/D) metal. Pt—ZSM—5, Zn—ZSM—5, etc. refer to the zeolite in the hydrogen form with the designated metal.

The process works without a H/D component. Presence of a H/D component may increase activity and/or selectivity. The examples show platinum metal helps. Other metals which can facilitate hydrogenation-dehydrogenation or olefin disproportionation, are the Fe or Pt metals of Group VIII of the Periodic Table, metals of Group IIb, titanium, vanadium, chromium, molybdenum, tungsten, rhenium and gallium, may be useful. (Chem. Rubber Handbook, 45th Ed., back cover).

## EXAMPLES

The examples were all run in a laboratory stainless steel downflow reactor 25 cm (10 inches long) having an internal diameter of 1.1 cm (7/16 inch). The catalyst was loaded into the lower 10 cm (4 inch) on a 2.5 cm (1.0 inch) bed of Vycor. The upper 12.5 cm (5 inches) was loaded with Vycor and served as a preheater. The catalysts were pretreated in air at 482°C (900°F) prior to use. If a metal was present, air calcination was followed by hydrogen reduction at 482°C (900°F). Material balances were made by collecting product in a liquid nitrogen-cooled trap and subsequent expansion into a precalibrated, constant volume glass system. Liquid and gas analysis were by gas chromatography.

The propane charge contained 99.5 wt% propane and 0.3—0.5 wt% propylene. Except for Example 19, which is not within the scope of this invention, none of the examples employed added hydrogen.

### Examples 1—15

The catalyst used in these examples was H—ZSM—5 having a silica-to-alumina ratio of 70:1, composited with alumina binder and extruded to form 1.6 mm (1/16 inch) diameter particles. The zeolite to binder weight ratio was 65:35. The same catalyst was used in Examples 1—15, without regeneration. The results are shown in Tables I, II, and III. The tests were all run at a pressure of 5600 kPa (800 PSIG).

TABLE I. CONVERSION OF PROPANE OVER H-ZSM-5

| EXAMPLE NO. | 1 | 2 | 3 | 4 | 5 |
|---|---|---|---|---|---|
| TEMPERATURE, °F | 651.00 | 676.00 | 676.00 | 675.00 | 675.00 |
| °C | 344.89 | 357.78 | 357.78 | 357.22 | 357.22 |
| WHSV | 1.00 | 1.00 | 1.10 | 1.10 | 1.00 |
| MATERIAL BALANCE | 100.99 | 101.37 | 98.19 | 106.25 | 117.87 |
| Time, hours | 5.30 | 28.60 | 52.60 | 120.10 | 144.10 |
| PRODUCT DISTRIBUTION, WT% | | | | | |
| C1 | 0.23 | 0.70 | 0.69 | 0.63 | 0.75 |
| C2 | 0.54 | 1.65 | 1.62 | 1.48 | 1.72 |
| C2= | 0.00 | 0.00 | 0.00 | 0.00 | 0.00 |
| C3 | 95.55 | 87.94 | 87.99 | 89.08 | 87.54 |
| C3= | 0.00 | 0.00 | 0.00 | 0.00 | 0.00 |
| ISO-C4 | 1.47 | 3.74 | 3.75 | 3.38 | 3.84 |
| N-C4 | 2.21 | 5.22 | 5.23 | 4.83 | 5.35 |
| C4= | 0.00 | 0.00 | 0.00 | 0.00 | 0.00 |
| ISO-C5 | 0.00 | 0.47 | 0.44 | 0.40 | 0.49 |
| N-C5 | 0.00 | 0.28 | 0.28 | 0.20 | 0.30 |
| WT% CONVERSION | 4.45 | 12.06 | 12.01 | 10.92 | 12.46 |
| TO C1-C4 | 4.45 | 11.31 | 11.29 | 10.32 | 11.67 |
| SELECTIVITY, WT% | | | | | |
| C1+C2 | 17.30 | 19.49 | 19.23 | 19.32 | 19.82 |
| ISO-C4 | 33.03 | 31.01 | 31.22 | 30.95 | 30.82 |
| N-C4 | 49.66 | 43.28 | 43.55 | 44.23 | 42.94 |
| C5'S | TRACE | 6.22 | 6.00 | 5.49 | 6.34 |
| C6+'S | 0.00 | 0.00 | 0.00 | 0.00 | 0.00 |

TABLE II CONVERSION OF PROPANE OVER H-ZSM-5

| EXAMPLE NO. | 6 | 7 | 8 | 9 | 10 |
|---|---|---|---|---|---|
| TEMPERATURE, °F | 675.00 | 700.00 | 700.00 | 700.00 | 700.00 |
| °C | 357.22 | 371.11 | 371.11 | 371.11 | 371.11 |
| WHSV | 1.00 | 1.00 | 1.00 | 1.10 | 1.00 |
| MATERIAL BALANCE | 101.21 | 111.13 | 110.22 | 102.53 | 117.87 |
| Time, hours | 172.90 | 192.10 | 216.10 | 292.60 | 312.10 |
| PRODUCT DISTRIBUTION, WT% | | | | | |
| C1 | 0.72 | 1.38 | 1.41 | 1.23 | 1.48 |
| C2 | 1.66 | 3.35 | 3.55 | 2.89 | 3.46 |
| C2= | 0.00 | 0.00 | 0.00 | 0.00 | 0.00 |
| C3 | 88.16 | 76.07 | 75.16 | 80.99 | 77.97 |
| C3= | 0.00 | 0.00 | 0.00 | 0.00 | 0.00 |
| ISO-C4 | 3.65 | 6.25 | 6.52 | 5.68 | 6.47 |
| N-C4 | 5.09 | 8.21 | 8.47 | 7.64 | 8.53 |
| C4= | 0.00 | 0.00 | 0.00 | 0.00 | 0.00 |
| ISO-C5 | 0.45 | 1.32 | 1.42 | 1.00 | 1.33 |
| N-C5 | 0.27 | 0.87 | 0.88 | 0.58 | 0.77 |
| C6+PAR+CYCLOPAR | 0.00 | 2.10 | 1.93 | 0.00 | 0.00 |
| C+6 AROMATICS | 0.00 | 0.46 | 0.64 | 0.00 | 0.00 |
| TOTAL C6+ PRODUCTS | 0.00 | 2.56 | 2.57 | 0.00 | 0.00 |
| WT% CONVERSION | 11.84 | 23.93 | 24.84 | 19.01 | 22.03 |
| TO C1-C4 | 11.12 | 19.19 | 19.94 | 17.43 | 19.93 |
| SELECTIVITY, WT% | | | | | |
| C1+C2 | 20.10 | 19.77 | 19.97 | 21.67 | 22.42 |
| ISO-C4 | 30.83 | 26.12 | 26.27 | 29.88 | 29.37 |
| N-C4 | 42.99 | 34.31 | 34.10 | 40.19 | 38.72 |
| C5'S | 6.08 | 9.15 | 9.26 | 8.31 | 9.53 |
| C6+'S | 0.00 | 10.70 | 10.35 | TRACE | TRACE |

TABLE III. CONVERSION OF PROPANE OVER H-ZSM-5

| EXAMPLE NO. | 11 | 12 | 13 | 14 | 15 |
|---|---|---|---|---|---|
| TEMPERATURE, °F | 700.00 | 700.00 | 701.00 | 701.00 | 675.00 |
| °C | 371.11 | 371.11 | 371.67 | 371.67 | 357.22 |
| WHSV | 1.00 | 1.10 | 1.00 | 1.10 | 1.00 |
| MATERIAL BALANCE | 108.13 | 103.20 | 105.56 | 104.06 | 106.29 |
| Time, hours | 336.10 | 360.10 | 384.10 | 456.10 | 480.10 |
| PRODUCT DISTRIBUTION, WT% | | | | | |
| C1 | 1.47 | 1.38 | 1.45 | 1.33 | 0.65 |
| C2 | 3.61 | 3.24 | 3.51 | 3.20 | 1.42 |
| C2= | 0.00 | 0.00 | 0.00 | 0.00 | 0.00 |
| C3 | 77.60 | 79.11 | 77.73 | 79.20 | 89.36 |
| C3= | 0.00 | 0.00 | 0.00 | 0.00 | 0.00 |
| ISO-C4 | 6.56 | 6.20 | 6.65 | 6.15 | 3.18 |
| N-C4 | 8.59 | 8.19 | 8.50 | 8.22 | 4.70 |
| C4= | 0.00 | 0.00 | 0.00 | 0.00 | 0.00 |
| ISO-C5 | 1.37 | 1.20 | 1.36 | 1.20 | 0.41 |
| N-C5 | 0.79 | 0.68 | 0.80 | 0.70 | 0.27 |
| WT% CONVERSION | 22.40 | 20.89 | 22.27 | 20.80 | 10.64 |
| TO C1-C4 | 20.24 | 19.01 | 20.12 | 18.90 | 9.95 |
| SELECTIVITY, WT% | | | | | |
| C1+C2 | 22.68 | 22.12 | 22.27 | 21.78 | 19.45 |
| ISO-C4 | 29.29 | 29.68 | 29.86 | 29.57 | 29.89 |
| N-C4 | 38.35 | 39.21 | 38.17 | 39.52 | 44.17 |
| C5'S | 9.64 | 9.00 | 9.70 | 9.13 | 6.39 |
| C6+'S | TRACE | TRACE | TRACE | TRACE | 0.00 |

Examples 16—21

The Pt—ZSM—50 catalyst used in these examples was prepared from an H—ZSM—50 zeolite having a silica-to-alumina ratio of 32:1. The zeolite was impregnated with tetrammine platinum dichloride using the incipient wetness technique. The final catalyst contained 2 wt% platinum. The pure zeolite (no binder) was meshed to 0.25 to 0.84 mm (20 × 60 mesh) particle size and charged to the reactor. Examples 16—21 used the same charge of catalyst, without regeneration. Example 19 shows added hydrogen is bad.

TABLE IV CONVERSION OF PROPANE OVER PT-ZSM-50

| EXAMPLE NO. | 16 | 17 | 18 | 19 | 20 | 21 |
|---|---|---|---|---|---|---|
| | | | | Comparison | | |
| TEMPERATURE, °F | 649.00 | 699.00 | 798.00 | 700.00 | 800.00 | 850.00 |
| °C | 342.78 | 370.56 | 425.56 | 371.11 | 426.67 | 454.44 |
| WHSV | 1.20 | 1.00 | 1.10 | 1.20 | 1.10 | 0.60 |
| $H_2$/HC MOL RATIO | 0.00 | 0.00 | 0.00 | 2.10 | 0.00 | 0.00 |
| MATERIAL BAL. | 92.80 | 103.81 | 105.88 | 90.86 | 91.68 | 103.89 |
| Time, hours | 39.50 | 63.20 | 87.20 | 93.70 | 113.60 | 118.60 |
| PRODUCT DISTRIBUTION, WT% | | | | | | |
| C1 | 0.05 | 0.05 | 0.41 | 0.23 | 0.25 | 0.54 |
| C2 | 0.57 | 1.25 | 3.80 | 0.89 | 2.66 | 1.05 |
| C2= | 0.00 | 0.00 | 0.00 | 0.00 | 0.00 | 0.03 |
| C3 | 91.89 | 89.07 | 79.77 | 98.81 | 83.95 | 93.26 |
| C3= | 0.00 | 0.00 | 0.00 | 0.00 | 0.00 | 0.39 |
| ISO-C4 | 3.44 | 4.17 | 5.24 | 0.07 | 4.80 | 1.90 |
| N-C4 | 3.62 | 4.80 | 7.27 | 0.00 | 6.14 | 2.83 |
| C4= | 0.00 | 0.00 | 0.00 | 0.00 | 0.00 | 0.00 |
| ISO-C5 | 0.34 | 0.50 | 0.89 | 0.00 | 0.41 | 0.00 |
| N-C5 | 0.08 | 0.16 | 0.47 | 0.00 | 0.22 | 0.00 |
| C6+PAR+CYCLOP | 0.00 | 0.00 | 0.98 | 0.00 | 0.38 | 0.00 |
| C6+ AROMATICS | 0.00 | 0 | 1.15 | 0.00 | 1.19 | 0.00 |
| TOTAL C6+ PRODS | 0.00 | 0.00 | 2.13 | 0.00 | 1.57 | 0.00 |
| WT% CONVERSION | 8.11 | 10.93 | 20.23 | 1.19 | 16.05 | 6.74 |
| TO C1-C4 | 7.69 | 10.27 | 16.72 | 1.19 | 13.86 | 6.74 |
| SELECTIVITY, WT% | | | | | | |
| C1+C2'S | 7.62 | 11.89 | 20.81 | 94.12 | 18.13 | 24.04 |
| ISO-C4 | 42.42 | 38.15 | 25.90 | 5.88 | 29.91 | 28.19 |
| N-C4 | 44.64 | 43.82 | 35.94 | 0.00 | 38.26 | 41.99 |
| C5'S | 5.18 | 6.04 | 6.77 | 0.00 | 3.93 | 0.00 |
| C6+'S | 0.00 | 0.00 | 10.58 | 0.00 | 9.78 | 0.00 |

Examples 22—24

The catalyst for Example 22 was prepared, charged and used in the same manner as the catalyst for Example 16 except that H—ZSM—5 having a silica-to-alumina ratio of 40 was used instead of H—ZSM—50. As in Example 16, no binder was used. The catalyst of Example 22 was used, without regeneration, for Examples 23 and 24.

TABLE V CONVERSION OF PROPANE OVER PT-ZSM-5

| EXAMPLE NO. | 22 | 23 | 24 |
|---|---|---|---|
| TEMPERATURE, °F | 725.00 | 601.00 | 625.00 |
| °C | 385.00 | 316.11 | 329.44 |
| WHSV | 1.10 | 1.20 | 0.50 |
| MATERIAL BALANCE | 104.73 | 91.27 | 111.43 |
| Time, hours | 22.50 | 29.30 | 26.70 |
| PRODUCT DISTRIBUTION, WT% | | | |
| C1 | 2.95 | 0.13 | 0.55 |
| C2 | 47.72 | 1.17 | 4.10 |
| C2= | 0.00 | 0.00 | 0.00 |
| C3 | 29.47 | 88.30 | 76.48 |
| C3= | 0.00 | 0.00 | 0.00 |
| ISO-C4 | 2.56 | 4.62 | 6.98 |
| N-C4 | 2.95 | 5.19 | 7.84 |
| C4= | 0.00 | 0.00 | 0.00 |
| ISO-C5 | 0.27 | 0.40 | 1.31 |
| N-C5 | 0.20 | 0.20 | 0.82 |
| C5= | 0.02 | 0.00 | 0.00 |
| C6+PAR+CYCLOPAR | 0.99 | 0.00 | 1.81 |
| C6+ AROMATICS | 12.87 | 0.00 | 0.11 |
| TOTAL C6+ PRODUCTS | 13.86 | 0.00 | 1.92 |
| WT% CONVERSION | 70.53 | 11.70 | 23.52 |
| TO C1-C4 | 56.19 | 11.11 | 19.47 |
| SELECTIVITY, WT% | | | |
| C1+C2 | 71.80 | 11.11 | 19.77 |
| ISO-C4 | 3.63 | 39.49 | 29.68 |
| N-C4 | 4.18 | 44.36 | 33.33 |
| C5'S | 0.69 | 5.13 | 9.06 |
| C6+'S | 19.05 | 0.00 | 8.16 |
| C4+'S | 28.20 | 88.89 | 80.23 |

Example 25

Example 1 is repeated but H—ZSM—11 is substituted for the H—ZSM—5 used in that example. The results are substantially the same.

Examples 26—28

In these examples the hydrogen form of the zeolite is composited with 35 wt% alumina and extruded to form 1.6 mm (1/16 inch) diameter extrudate. Platinum was impregnated on the catalyst using the incipient wetness technique using chloroplatinic acid in a concentration to provide the desired content of platinum. The catalyst of Example 26 was made with H—ZSM—22 having a silica-to-alumina ratio of 82 and the catalyst had a Pt content of 0.57 wt%. The catalyst of Example 27 was made with H—ZSM—23 having a silica-to-alumina ratio of 115, and the catalyst had a platinum content of 0.05 wt%. The catalyst of Example 28 was made with H—ZSM—35 having a silica-to-alumina ratio of 13, and the catalyst had a platinum content of 0.59 wt% platinum.

All three catalysts were tested as described in Example 1 using 5600 kPa (800 psig) pressure and 1 WHSV. The results are shown in Table VI.

TABLE VI CONVERSION OF PROPANE OVER ZEOLITES ZSM-22, ZSM-23, ZSM-35

| EXAMPLE NO. | 26 | 27 | 28 |
|---|---|---|---|
| CATALYST | Pt-ZSM-22 | Pt-ZSM-23 | Pt-ZSM-35 |
| TEMPERATURE, °F | 700 | 748 | 750 |
| °C | 371 | 398 | 399 |
| WT% CONVERSION | 10 | 11 | 8 |
| SELECTIVITIES | | | |
| C1+C2 | 40 | 35 | 38 |
| ISO-C4 | 16 | 15 | 6 |
| N-C4 | 19 | 20 | 12 |
| C5+'S | 25 | 30 | 42 |

Example 29

In this example the catalyst was prepared from 26 grams $NH_4$—ZSM—5 having a silica-to-alumina ratio of 40. This was exchanged once with a mixture of 10 grams $NH_4NO_3$ and 3 g $ZnCl_2$ in 100 cc of water at 38°C (100°F) for 17 hours. It was rinsed and dried at 38°C (100°F) for 22 hours. It was meshed to 0.25 to 0.84 mm (20 × 60 mesh) and calcined in air at 538°C (1000°F) for 12 hours. The resultant binder-free catalyst had 0.38 wt% Zn.

The catalyst was loaded into the reactor and tested as in Example 1 at 5600 kPa (800 psig) and 0.4 WHSV. The conversion was 16 wt%, with the following selectivities:

| | |
|---|---|
| C1 + C2 | 21 wt% |
| ISO—C4 | 32 wt% |
| N—C4 | 40 wt% |
| C5 + 'S | 7 wt% |

**Claims**

1. A process for producing butanes from propane characterized by contacting a propane feed in the absence of added hydrogen with a catalyst comprising a crystalline zeolite having a silica-to-alumina ratio of at least 12 and a Constraint Index of 1 to 12 at a temperature of 260 to 482°C (500 to 900°F), a pressure of 450 to 10400 kPa (50 to 1500 psig), and a WHSV of 0.1 to 10.

2. The process of claim 1 further characterized in that the catalyst has a hydrogenation-dehydrogenation component.

3. The process of claim 2 further characterized in that the hydrogenation-dehydrogenation component is a platinum group metal.

4. The process of any preceding claim further characterized in that the zeolite is selected from the group of ZSM—5, ZSM—11, ZSM—12, ZSM—22, ZSM—23, ZSM—35 and ZSM—50.

**Patentansprüche**

1. Verfahren zur Herstellung von Butanen aus Propan, gekennzeichnet durch den Kontakt der Propanzufuhr ohne zugesetzten Wasserstoff mit einem Katalysator, der einen kristallinen Zeolith mit einem Siliciumdioxid/Aluminiumoxid-Verhältnis von mindestens 12 und einem Zwangsindex von 1 bis 12 umfaßt, bei einer Temperatur von 260 bis 482°C (500 bis 900°F), einem Druck von 450 bis 10400 kPa (50 bis 1500 psig) und einer stündlichen Gewichts-Raum-Geschwindigkeit von 0,1 bis 10.

2. Verfahren nach Anspruch 1, weiterhin dadurch gekennzeichnet, daß der Katalysator eine Hydrierungs/Dehydrierungs-Komponente aufweist.

3. Verfahren nach Anspruch 2, weiterhin dadurch gekennzeichnet, daß die Hydrierungs/Dehydrierungs-Komponente ein Metall der Platingruppe ist.

4. Verfahren nach einem der vorstehenden Ansprüche, weiterhin dadurch gekennzeichnet, daß der Zeolith aus der Gruppe von ZSM—5, ZSM—11, ZSM—12, ZSM—22, ZSM—23, ZSM—35 und ZSM—50 ausgewählt ist.

**Revendications**

1. Un procédé de production de butanes à partir de propane, caractérisé en ce qu'une charge de propane est mise au contact, en l'absence d'hydrogène supplémentaire, d'un catalyseur comprenant une zéolite cristalline ayant un rapport silice/alumine d'au moins 12 et un indice de contrainte compris entre 1 et 12, à une température comprise entre 260 et 482°C (500 et 900°F), une pression de 450 à 10 400 kPa (50 à 1 500 psig) et une vitesse spatiale horaire pondérale de 0,1 à 10.

2. Le procédé selon la revendication 1, caractérisé en outre en ce que le catalyseur contient un constituant d'hydrogénation/déshydrogénation.

3. Le procédé selon la revendication 2, caractérisé en outre en ce que le constituant d'hydrogénation/déshydrogénation est un métal du groupe du platine.

4. Le procédé selon l'une quelconque des revendications précédentes, caractérisé en outre en ce que la zéolite est choisie dans le groupe comprenant ZSM—5, ZSM—11, ZSM—12, ZSM—22, ZSM—23, ZSM—35 et ZSM—50.